(19) Europäisches Patentamt
European Patent Office
Office européen des brevets

(11) Numéro de publication: **0 004 257**
**B1**

(12) # FASCICULE DE BREVET EUROPEEN

(45) Date de publication du fascicule du brevet: **20.01.82**

(21) Numéro de dépôt: **79870005.0**

(22) Date de dépôt: **07.03.79**

(51) Int. Cl.³: **C 07 C 149/415, C 07 D 335/16**

(54) **Nouveau dérivé de benzonitrile, son procédé de préparation et son application.**

(30) Priorité: **09.03.78 FR 7806741**

(43) Date de publication de la demande:
**19.09.79 Bulletin 79/19**

(45) Mention de la délivrance du brevet:
**20.01.82 Bulletin 82/3**

(84) Etats contractants désignés:
**BE CH DE IT NL SE**

(56) Documents cités:
**GB - A - 951 651**
**GB - A - 1 447 031**
**GB - A - 1 447 032**
**US - A - 3 711 513**

**CHEMICAL ABSTRACTS, vol 52, nº 9, 10 mai 1958 Colombus, Ohio (USA)**

**G. LEANDRI et al. "Synthesis and anti-bacterial activity of aromatic amidoxime and diazafuran derivatives" abstract nº 7291h—7292c**

(73) Titulaire: **SANOFI, société anonyme**
**40, Avenue George V**
**F-75008 Paris (FR)**

(72) Inventeur: **Pigerol, Charles**
**8, rue Carnot**
**F-93400 Saint-Ouen (FR)**
Inventeur: **Bouisset, Michel**
**Immeuble "Le Grognard" Avenue de la Libération**
**F-04200 Sisteron (FR)**
Inventeur: **Chignac, Michel**
**"Le Gand" Avenue du Lac**
**F-04200 Sisteron (FR)**
Inventeur: **Grain, Claude**
**Villa "Les Olivettes" La Croix du Sud**
**F-04290 Volonne (FR)**

(74) Mandataire: **Delaunois, Jacques et al,**
**c/o S.A. LABAZ N.V. Avenue de Béjar 1**
**B-1120 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 004 257 B1

# Nouveau dérivé de benzonitrile, son procédé de préparation et son application

La présente invention se rapporte d'une manière générale à un nouveau dérivé de benzonitrile utile comme produit de départ dans la synthèse de la chloro-2 thioxanthone.

La présente invention se rapporte plus précisément au (chloro-4' phénylthio)-2 benzonitrile de formule:

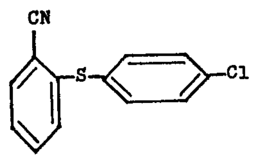

L'invention se rapporte également au procédé de préparation du composé de formule I, ledit procédé étant caractérisé en ce que l'on fait réagir dans un solvant organique un dérivé de métal alcalin du chloro-4 thiophénol avec du chloro-2 benzonitrile.

L'invention se rapporte enfin à l'application du composé I à la préparation de la chloro-2 thioxanthone, consistant à hydrolyser, en milieu acide ou basique, le (chloro-4' phénylthio)-2 benzonitrile, pour obtenir l'acide (chloro-4' phénylthio)-2 benzoïque, puis à cycliser cet acide par déshydratation pour donner la chloro-2 thioxanthone.

Le (chloro-4' phénylthio)-2 benzonitrile est un produit nouveau, et est revendiqué en tant que tel. Il permet d'obtenir par une voie nouvelle et avantageuse la chloro-2 thioxanthone, produit connu comme photoinitiateur pour la réticulation des résines synthétiques, notamment des résines acryliques, ainsi que décrit dans le brevet américain No. 3.876.519, ou comme intermédiaire pour la préparation de produits pharmaceutiques, ainsi que décrit dans le brevet français No. 1.352.102.

Le brevet français No 1.173.489 décrit un procédé de préparation de la chloro-2 thioxanthone, consistant à faire réagir un dérivé de métal alcalin du chloro-4 thiophénol avec un acide halogéno-2 benzoïque pour donner l'acide (chloro-4' phénylthio)-2 benzoïque, puis à cycliser par déshydratation ce dernier acide pour obtenir la chloro-2 thioxanthone.

Ce procédé se différencie du procédé de l'invention par le fait que l'on part directement d'acide halogéno-2 benzoïque pour préparer l'acide (chloro-4' phénylthio)-2 benzoïque, sans passer donc par le (chloro-4' phénylthio)-2 benzonitrile, objet de l'invention.

Ce procédé, apparement plus direct, ne pourrait toutefois pas être appliqué à l'échelle industrielle car non seulement il donne des rendements faibles, mais il implique l'utilisation de produits de départ assez onéreux.

Ainsi, la réaction d'un dérivé de métal alcalin du chloro-4 thiophénol avec l'acide chloro-2 benzoique conduit à l'acide (chloro-4' phénylthio)-2 benzoïque avec un redement ne dépassant pas 20 à 30%, soit un rendement trop bas pour pouvoir envisager une application industrielle de cette réaction. Quant à la réaction avec l'acide bromo-2 benzoïque, elle donne un meilleur rendement, pouvant atteindre 50%, mais celui-ci est encore trop bas pour compenser le prix plus élevé de ce réactif. Enfin, la réaction avec l'acide iodo-2 benzoïque donne un rendement compris entre 40 et 50%, soit un rendement trop faible pour une application industrielle rentable. De plus, l'acide iodo-2 benzoïque est relativement cher et assez rare sur le marché des produits chimiques.

Par contre, la réaction impliquant le passage par le (chloro-4' phénylthio)-2 benzonitrile, le produit selon l'invention, permet d'obtenir la chloro-2 thioxanthone avec de très bons rendements, approchant 80—85%, et en partant de produits de départ bon marché et largement disponibles sur le marché des produits chimiques.

On connaît également un procédé de préparation d'un composé analogue à la chloro-2 thioxanthone, à savoir la chloro-1 thioxanthone, produit de départ de l'hycanthone.

D'aprés le brevet américain No 3.711.513, il est connu de préparer la chloro-1 thioxanthone en faisant réagir du dichloro-2,6 benzonitrile avec un sel de métal alcalin du thiophénol pour obtenir de la chloro-2 phénylthio-6 benzonitrile, laquelle est transformée au moyen d'acide polyphosphorique en chloro-1 imino-9 thioxanthène, ce dernier produit étant hydrolysé en chloro-1 thioxanthone.

Mis à part les réactifs utilisés et le produit final obtenu, ce procédé se différencie essentiellement de celui de l'invention par le fait que l'on n'hydrolyse pas la chloro-2 phénylthio-6 benzonitrile, mais que l'on cyclise directement ce produit en chloro-1 imino-9 thioxanthène, et c'est ce dernier produit qui est hydrolysé en chloro-1 thioxanthone.

Cette différence est importante et il faut remarquer que le procédé décrit ci-dessus est très peu rentable et ne pourrait pas être utilisé à l'échelle industrielle : son rendement global en chloro-1 thioxanthone n'atteint pas en effet 15%.

On connait enfin, d'après le brevet britannique No 1.447.031, un procédé de préparation de dérivés de thioxanthone partant d'acide phénylthiotéréphtalique, lequel est préparé à partir de nitrotéréphtalonitrile.

Ce procédé se différencie essentiellement du procédé de l'invention par l'utilisation d'un dérivé nitré (nitrotéréphtalonitrile) au lieu d'un dérivé chloré (chlor-2 benzonitrile).

Le nitrotéréphtalonitrile est un produit qui n'est pas commercialisé et qui doit être préparé préalablement à la mise en oeuvre du procédé du brevet britannique, ce qui alourdit le prix de revient de ce procédé.

Or, il est connu que le chloro-2 benzonitrile est un produit courant et, de plus, bon marché.

Enfin, la Demanderesse a préraré le composé de l'invention en s'inspirant de l'enseignement du brevet britannique No 1.447.031, donc en partant de nitro-2 benzonitrile: un rendement de 37% a été obtenu, que l'on peut comparer au 96% obtenu à partir de chloro-2 benzonitrile. De plus, le nitro-2 benzonitrile est un produit de 20 à 25 fois plus cher que le chloro-2 benzonitrile.

Il ne serait donc pas possible d'utiliser à l'échelle industrielle le procédé du brevet britannique ci-dessus.

Les Exemples ci-dessous illustrent, d'une manière non limitative, le procédé de préparation du composé selon l'invention, ainsi que l'application de ce comosé à la préparation de la chloro-2 thioxanthone.

Exemple 1

*Préparation du (chloro-4' phénylthio)-2 benzonitrile*

Dans un réacteur en verre de deux litres, équipé d'un réfrigérant, d'un agitateur, d'une ampoule à brome et d'une arrivée d'azote, on introduit 200 g de méthanol et on ajoute peu à peu 23 g (1 at. g.) de sodium. Lorsque tout le sodium est consommé, on élimine par distillation 80 g de méthanol. On refroidit le milieu réactionnel et on introduit en une seule fois 144,5 g (1 mole) de chloro-4 thiophénol. On chauffe le mélange à reflux pendant 0,25 heure, puis on refroidit à 80°C et on ajoute rapidement un solution de 133,5 g (0,97 mole) de chloro-2 benzonitrile dans 300 g de toluène, et enfin 94,5 g de N,N-diméthylformamide. On distille ensuite environ 250 g du mélange azéotropique toluène-méthanol, puis 80 g de toluène. On chauffe le milieu réactionnel pendant 1,5 heure à 125—130°C puis on le refroidit à 60°C et on introduit successivement 420 g de toluène et 200 g d'eau.

On décante le mélange à 50°C et on lave la phase organique avec 200 g d'une solution aqueuse de soude à 5%, puis deux fois avec 200 g, la température étant maintenue à 50°C.

On élimine le toluène et on recueille 226,44 g de (chloro-4' phénylthio)-2 benzonitrile, ce qui correspond à un rendement de 95% en produit brut.

Le produit brut fond à 82°C et, après recristallisation dans l'éthanol, à 87°C.

En fait, il n'est pas nécessaire d'isoler le (chloro-4' phénylthio)-2 benzonitrile car la solution toluénique de ce composé peut être utilisée telle quelle pour la synthèse de l'acide (chloro-4' phenylthio)-2 benzoïque, lequel est ensuite transformé en chloro-2 thioxanthone.

Exemple 2

*Préparation du chloro-4' phénylthio)-2 benzonitrile*

On introduit dans un réacteur de 250 ml équipé d'un appareil de Dean Stark et d'une arrivée d'azote 28,9 g (0,2 mole) de chloro-4 thiophénol, une solution de 8,2 g (0,205 mole) de soude dans 30 g d'eau. La température du mélange s'élève à 50—60°C et on maintient cette température pendant 0,5 heure, puis on introduit 30 ml de benzène, On élimine alors environ la moitié de l'eau par distillation azéotropique du mélange eau-benzène. On ajoute 40 ml de diméthylsulfoxyde et on élimine par distillation la totalité de l'eau et du benzène.

A la température de 50°C, on introduit 27,5 g (0,2 mole) de chloro-2 benzonitrile et on porte la température à 75 ± 5°C pendant 5 heures. On extrait ensuite le mélange avec 250 ml de toluène et on lave la phase organique à l'eau pour éliminer le chlorure de sodium, et on élimine le solvant par évaporation sous vide.

On obtient 47,2 g de (chloro-4' phénylthio)-2 benzonitrile, ce qui correspond à un rendement de 96%.

Exemple 3

*Préparation du (chloro-4' phénylthio)-2 benzonitrile*

On introduit dans un réacteur de deux litres 300 g de méthanol puis, sous courant d'azote, 85,05 g (1,57 mole) de méthylate de sodium. On élimine par distillation 120 g de méthanol et on refroidit à 30°C, puis on introduit en une seule fois 216,75 g (1,49 mole) de chloro-4 thiophénol. On chauffe le mélange à reflux pendant 0,5 heure puis on laisse la température descendre jusqu'à 70°C et on introduit peu à peu une solution de 200,25 g (1,45 mole) de chloro-2 benzonitrile dans 450 g de toluène, puis 150 g de N,N-diméthylformamide.

On distille ensuite environ 530 g du mélange azéotropique méthanol-toluène, puis environ 95 d ge toluène.

On chauffe alors le mélange réactionnel au reflux pendant 1,5 heure puis on laisse la température descendre jusqu'à 60°C et on introduit 500 g de toluène et 300 g d'eau.

On décante la phase aqueuse et on effectue un lavage de la phase toluénique par une solution de 15 g de soude dans 280 g d'eau, la température étant maintenue à 50—55°C. On ajoute à nouveau 150 g de toluène et on effectue encore deux lavages avec deux fois 300 g d'eau, puis un dernier lavage avec 7,5 g d'acide acétique à 90% dans 300 ml d'eau. On obtient, après évaporation du toluène sous pression réduite, 357 g de (chloro-4' phénylthio)-2 benzonitrile, ce qui correspond à un rendement de 97%.

Exemple 4

*Préparation du (chloro-4' phénylthio)-2 benzonitrile*

On mélange 144,5 g (1 mole) de chloro-4 thiophénol avec 300 g de méthoxy-2 éthanol et on introduit peu à peu 24,5 g (1,05 mole) de sodium. Lorsque tout le sodium est consommé, on ajoute 137,5 g (1 mole) de chloro-2 benzonitrile et on chauffe le mélange à reflux (118°C).

On isole le produit comme indiqué dans les Exemples précédents et l'on obtient 207,2 g de (chloro-4' phénylthio)-2 benzonitrile, ce qui correspond à un rendement de 83,9%.

Exemple 5

*Préparation de l'acide (chloro-4' phénylthio)-2 benzoïque*

Dans un réacteur de trois litres équipé d'un agitateur, d'une ampoule à brome et d'un réfrigérant, on introduit 1100 g d'une solution de toluène contenant 355 g (1,44 mole) de (chloro-4' phénylthio)-2 benzonitrile, préparé comme à l'Exemple 1. On élimine le toluène par distillation sous pression atmosphérique puis sous pression réduite. On introduit ensuite en 15 minutes environ une solution d'acide sulfurique à 65% obtenue à partir de 1255 g d'acide sulfurique à 94% et de 560 g d'eau. On porte la température du milieu réactionnel à 125—130°C pendant 8h et on verse le mélange dans un bécher de 5 litres. On refroidit et on introduit deux litres d'eau en 10 minutes environ. On refroidit à 20 ± 5°C et on maintient le mélange à cette température pendant 0,5 heure. On essore ensuite le précipité et on le lave à l'eau jusqu'à élimination des ions sulfates. le solide est placé dans un ballon muni d'un réfrigérant à reflux; on ajoute 730 g de méthanol et on chauffe pendant 1 heure. On refroidit à 20°C, on maintient le mélange sous agitation pendant 1 heure à la même température puis on essore le précipité. On le rince avec 180 g de méthanol et on le sèche à l'étuve. On obtient 336,5 g d'acide (chloro-4' phénylthio)-2 benzoïque fondant à 240°C, ce qui correspond à un rendement de 88%.

Exemple 6

*Préparation de l'acide (chloro-4' phénylthio)-2 benzoïque*

On introduit dans un réacteur de 3 litres équipé d'un réfrigérant 682,5 g d'une solution de toluène contenant 200 g (0,815 mole) de (chloro-4' phénylthio)-2 benzonitrile, préparé comme dans un des Exemples 1 à 4, et on distille le toluène sous pression atmosphérique. Lorsque la distillation est terminée, on refroidit à 100°C et on ajoute 400 g d'eau puis on poursuit l'élimination du toluène par distillation azéotropique du mélange eau-toluène.

On refroidit le mélange à 70°C et on ajoute successivement 400 ml de méthoxy-2 éthanol, 100 g de soude et 40 ml d'eau. On chauffe le mélange au reflux pendant 6 heures puis on le refroidit à 80°C. On introduit sous agitation un mélange de 260 ml d'acide chlorhydrique à 36% et 200 g d'eau. On essore le précipité à chaud, on le lave à l'eau jusqu'à neutralité et on le sèche. On obtient l'acide (chloro-4' phénylthio)-2 benzoïque avec un rendement quantitatif.

Le produit final ne renferme que des traces, en quantité inférieure à 0,5% de (chloro-4' phénylthio)-2 benzamide, déterminée par chromatographie sur couche mince.

Exemple 7

*Préparation de l'acide (chloro-4' phénylthio)-2 benzoïque*

On chauffe à reflux pendant 8h à 130°C dans un réacteur équipé d'un réfrigérant, un mélange de 245,7 g (1 mole) de (chloro-4' phénylthio)-2 benzonitrile, préparé comme dans un des Exemples 1 à 4, avec 701,5 g d'acide sulfurique à 70% (5 moles) et 245 ml d'acide acétique à 90%.

On refroidit ensuite le mélange à ± 5°C et on introduit peu à peu trois litres d'eau. Après avoir laissé le mélange pendant 0,5 h à cette température, on filtre le précipité et on le rince avec 180 g de méthanol.

On obtient 242,2 g d'acide (chloro-4' phénylthio)-2 benzoïque fondant à 240°C, ce qui correspond à un rendement de 91,5%.

Le produit final ne renferme qu'une quantité inférieure à 1% de (chloro-4' phénylthio)-2 benzamide.

**Revendications**

1. Chloro-4' phénylthio)-2 benzonitrile.

2. Procédé de préparation du (chloro-4' phénylthio)-2 benzonitrile charactérisé en ce que l'on fait réagir dans un solvant organique un dérivé de métal alcalin du chloro-4 thiophénol avec du chloro-2 benzonitrile.

3. Procédé de préparation selon la Revendication 2 caractérisé en ce que le solvant organique est la N,N-diméthylformamide.

4. Procédé de préparation selon la Revendication 2 caractérisé en ce que le solvant organique est la diméthylsulfoxyde.

5. Procédé de préparation selon la Revendication 2 caractérisé en ce que le solvant organique est le méthoxy-2 éthanol.

6. Procédé de préparation selon la Revendication 2 caractérisé en ce que le métal alcalin est le sodium.

7. Application du (chloro-4' phénylthio)-2 benzonitrile à la préparation de la chloro-2 thioxanthone caractérisée en ce que l'on hydrolyse, en milieu acide ou basique, le (chloro-4' phénylthio)-2 benzonitrile, puis en ce que l'on cyclise par déshydratation, l'acide (chloro-4' phénylthio)-2 benzoïque ainsi obtenu pour former la chloro-2 thioxanthone.

**Claims**

1. 2-(4'-chloro-phenylthio)-benzonitrile.

2. Process for preparing 2-(4'-chloro-phenylthio)-benzonitrile whereby an alcali metal derivative of 4-chloro-thiophenol is reacted in an organic solvent with 2-chloro-benzonitrile.

3. Process according to Claim 2 whereby the organic solvent is N,N-dimethylformamide.

4. Process according to Claim 2 whereby the organic solvent is dimethylsulphoxide.

5. Process according to Claim 2 whereby the

organic solvent is 2-methoxy-ethanol.

6. Process according to Claim 2 whereby the alcali metal is sodium.

7. Use of 2-(4'-chloro-phenylthio)-benzonitrile for preparing 2-chlorothioxanthone whereby 2-(4'-chloro-phenylthio)-benzonitrile is hydrolyzed in an acid or base medium to give 2-(4'-chloro-phenylthio)-benzoic acid which is cyclized by dehydration to give 2-chlorothioxanthone.

**Patentansprüche**

1. 2-(4'-Chlor-phenylthio)-benzonitril.

2. Verfahren zur Herstellung von 2-(4'-Chlor-phenylthio)-benzonitril, dadurch gekennzeichnet, dass man in einem organischen Lösungsmittel ein Alkalimetallderivat des 4-Chlor-thiophenols mit 2-Chlor-benzonitril reagieren lässt.

3. Herstellungsverfahren nach Anspruch 2, dadurch gekennzeichnet, dass das organische Lösungsmittel N,N-Dimethylformamid ist.

4. Herstellungsverfahren nach Anspruch 2, dadurch gekennzeichnet, dass das organische Lösungsmittel Dimethyl-sulfoxid ist.

5. Herstellungsverfahren nach Anspruch 2, dadurch gekennzeichnet, dass das organische Lösungsmittel 2-Methoxy-äthanol ist.

6. Herstellungsverfahren nach Anspruch 2, dadurch gekennzeichnet, dass das Alkalimetall Natrium ist.

7. Verwendung von 2-(4'-Chlor-phenylthio)-benzonitril zur Herstellung von 2-Chlor-thioxanthon, dadurch gekennzeichnet, dass man das 2-(4'-Chlor-phenylthio)-benzonitril in saurem oder basischem Milieu hydrolisiert, worauf man die so erhaltene 2-(4'-Chlor-phenylthio)-benzoesäure dehydratisiert, un das 2-Chlor-thioxanthon zu bilden.